Europäisches Patentamt

European Patent Office

Office européen des brevets

(11) Publication number: **0 281 812**
**A1**

# EUROPEAN PATENT APPLICATION

(21) Application number: **88102370.9**

(22) Date of filing: **18.02.88**

(51) Int. Cl.⁴: **A61K 33/30** , A61K 33/06 ,
//(A61K33/30,33:04,31:60,31:19,
31:195),(A61K33/06,33:04,
31:60,31:19,31:195)

Claims for the following Contracting States: GR
+ ES.

(30) Priority: **18.02.87 US 16113**
**11.02.88 US 149177**

(43) Date of publication of application:
**14.09.88 Bulletin 88/37**

(84) Designated Contracting States:
**AT BE CH DE ES FR GB GR IT LI LU NL SE**

(71) Applicant: **MILOR SCIENTIFIC, LTD.**
**5630 Lake Mendota Drive**
**Madison Wisconsin 53705(US)**

(72) Inventor: **Schinitski, Michael R.**
**5630 Lake Mendota Drive**
**Madison Wisconsin 53705(US)**

(74) Representative: **Kraus, Walter, Dr. et al**
**Patentanwälte Kraus, Weisert & Partner**
**Thomas-Wimmer-Ring 15**
**D-8000 München 22(DE)**

(54) **Composition for treatment of acne.**

(57) A method is described for treating acne using a keratolytic agent, an astringent, and an anti-inflammatory agent either sequentially or in combination. Compositions comprising the active ingredients are also described.

EP 0 281 812 A1

## COMPOSITION FOR TREATMENT OF ACNE

### Background and Summary of the Invention

This invention relates to a topical therapeutic preparation for use in the treatment of acne. More particularly, this invention relates to compositions employing an anti-inflammatory agent, an astringent, and a keratolytic agent as active ingredients. It is especially useful for the treatment of whiteheads, Plugged sebaceous follicles with enlarged lining cells and filled with sebum, keratin and various bacteria.

It has been found that mild anti-inflammatory agents can be used together with a keratolytic agent and an astringent to produce a therapeutically effective acne preparation. Most acne treatment preparations contain anti-bacterial agents to treat the development and spread of acne bacteria. In contrast, the present invention is based on the premise that most acne problems derive not from the bacteria infection itself but instead result from the host's own inflammatory response to acne bacteria.

In plugged follicles characteristic of acne infections, bacteria can proliferate and release enzymes which contribute to the break-down of the follicular wall. The most common reason for the severe spreading of acne, however, is not the acne bacteria itself but rather the body's response to the bacteria. The degradation of the epidermal lining beneath the surface of the skin allows certain host cells to perceive the presence of the bacteria and to mount an inflammatory reaction which is characterized by the proliferation of polymorphonuclear leukocytes, lymphocytes and macrophages. Polymorphonuclear leukocytes and macrophages release proteolytic enzymes which, although having a limited effect on the bacteria, are more damaging to the integrity of the follicular lining than are the enzymes of the bacteria themselves. This inflammatory response contributes to the rupture of the follicle beneath the epidermis, thereby spreading the inflammatory response beneath the skin surface. This is why, despite impeccable skin care on the surface of the skin, acne continues to spread beneath the skin. Although the bacteria play a role in the spread of acne and the associated inflammation, by far the most devastating effect relative to the destruction of the follicle is the host's own immune system. Thus, it is the enzymes produced by the host that are the primary cause of the irritation and spread of acne and not the mere presence of acne bacteria.

For severe cases of acne vulgaris or other inflammatory lesions of the skin, current treatment compositions and methods rely primarily on the use of antibiotics and/or comedolytics, either alone or in combination to treat the acne bacteria (Corynebacterium acnes or C. acnes). Binnock, Skin Diseases: Diagnosis and Treatment in Clinical Practice, 1982. Oral tetracycline is often prescribed for severe acne. Most topical acne preparations contain benzoyl peroxide which, according to Kligman (U.S. Patent No. 4,318,907), is a drug of choice because it is "anti-microbial and therefore suppresses the acne bacillis, an organism which has an important causal role in acne vulgaris." Kligman's U.S. Patents 4,318,907 and 4,355,028 embody a combination of benzoyl peroxide and salicylic acid for application either simultaneously or sequentially for the purpose of eliminating the acne bacteria. Salicylic acid, a keratolytic agent, is used to enhance the penetration of the keratolytic/anti-bacterial agent, benzoyl peroxide. Notwithstanding these and other anti-bacterial treatment preparations, however, many patients still experience the spread of acne.

Current acne therapy advises against the use of strong anti-inflammatory agents. For example, topical use of fluorinated steroids or even steroids taken internally for other causes is known to aggravate acne or to cause a severe form of acne called "steroid acne". Kligman, 1982; Vasarinsh. Clinical Dermatology, Diagnosis and Therapy of Common Skin Diseases, Butterworth's Boston and London 1982. When a strong anti-inflammatory agent such as a steroid is used, there often is an initial improvement; however, the acne bacteria will continue to spread because of lack of host resistance. Without host recognition of the presence of the proliferating bacteria beneath the skin due to a steroid-suppressed inflammatory response, there is nothing to curtail the spread of bacteria. The bacteria, therefore, can spread under the skin to colonize many more follicles leading to the development of the severe acne vulgaris associated with the use of steroids.

Previous inventions have taught the use of milder, non-fluorinated, anti-inflammatory agents such as acetylsalicylates or esters thereof. U.S. Patents No. 4,126,681 and 4,244,948 are directed to the treatment of inflammatory lesions of the skin, including acne, and specifically address temporary alleviation of inflammation. However, acetylsalicylic acid and esters thereof are the only active ingredients employed in these formulations, with the rest of the formulation merely providing a vehicle to enhance absorption of the anti-inflammatory agent into the skin. It is clear that more than just an anti-inflammatory agent is needed to control the cause and spread of acne. It is also important to unplug the follicles in which the sebum, keratin, and proliferating bacteria accumulate and expand, to alleviate the pressure that builds up against the subcutaneous follicular walls. If the follicle cannot be unplugged, it will rupture beneath the skin.

The present invention is based on the discovery that a mild anti-inflammatory agent can be combined with a keratolytic agent which facilitates the opening and emptying of the contents of the engorged follicle onto the skin surface. This minimizes the chance that follicles will rupture beneath the skin which is inevitable if the comedo is not opened. Astringent agents aid in the contraction and emptying of the follicle and further lead to contraction of the large pores associated with acne-prone skin. Skin bacteria are less likely to enter such contracted pores, thus diminishing the topical spread and occurrence of other acne lesions.

The present invention is unique in that it is based on the premise that the C. acnes bacteria are normally present on skin as well as in the plugged follicles characteristic of acne. Because C. acnes are a part of the normal skin flora and do not themselves cause acne, there is no need to use active ingredients having anti-bacterial characteristics.

It is therefore an object of this invention to provide an improved composition and method for treating acne.

It is another object of this invention to provide a composition for treating acne that is not irritating to normal or sensitive skin and does not promote the uncomfortable drying sensation associated with other acne treatments.

Another object of this invention is to provide topical therapeutic acne treatment formulations having improved properties for alleviating skin damage caused by the host's inflammatory reaction to the spread of acne.

It is still a further object of this invention to provide a novel therapeutic approach to treatment of acne condition, one which does not rely for efficacy on the susceptibility of the acne-causing bacteria to a topical antibiotic-containing preparation.

Detailed Description of the Invention

The present invention is directed to a composition for the treatment of acne comprising an anti-inflammatory agent, an astringent, and a keratolytic agent. The keratolytic or comedolytic agent is used to disintegrate the keratin plug and to aid in peeling the skin down to the follicle so that the follicle is opened to the skin's exterior. The astringent is used to assist in the complete emptying of the follicle by causing contraction of the lining cells of the follicle. The astringent is further effective to reduce swelling in the edematous area immediately surrounding the affected follicle. A mild anti-inflammatory agent is also employed to suppress the body's damaging inflammatory reaction that occurs when the irritating contents of the follicle are discharged onto the surface of the skin. It is these three mechanisms which constitute the rationale of the therapeutic treatment scheme of the present invention and which dictate the three types of ingredients selected for the formulation and method of treatment of this invention.

The invention features the use of keratolytic and comedolytic agents to open the plugged skin follicle. The particular keratolytic agent employed is not critical and substantially any non-toxic keratolytic or comedolytic may be used to open the skin follicle. Suitable keratolytic agents include salicylic acid, retinoic acid, resorcinol, tretonin, sulfur, benzoic acid, urea and benzoyl peroxide. Such agents can be used alone or in combination. Salicylic acid is preferred because it produces minimal irritation to the skin and can be used at higher concentrations --up to 30% by weight of the topical formulation. Although salicylic acid has been used as a keratolytic agent in formulations for the treatment of acne (see, e.g., Kligman's Patent No. 4,318,907), the concentration of salicylic acid used was only 3%-7% by weight. Salicylic acid could not be used in higher concentrations because, when used in combination with the antimicrobial/keratolytic benzoyl peroxide, it caused excessive irritation at concentrations as low as 5%. Moreover, salicylic acid was used in Kligman's invention to facilitate the entrance of the benzoyl peroxide into the comedo, thereby increasing the permeability of benzoyl peroxide into the skin. An increased tissue concentration of benzoyl peroxide met with a corresponding increase in the efficacy of the acne preparation described by Kligman.

This use of salicylic acid is in marked contrast to the use of salicylic acid in the present invention whereby salicylic acid or other keratolytic agents are used to unclog skin follicles and to permit their contents to be released to the outside (skin surface). The astringent and mild anti-inflammatory agent cooperate to limit and/or to alleviate the inflammatory reaction which can be caused by the discharge of corrosive follicular contents (free fatty acids, bacteria and bacterial extracellular enzymes and waste products) onto the skin's surface. The preferred keratolytic agent, salicylic acid, may be used in the present invention at a relatively high dose by weight (8% to 30%), because it is not used with irritating chemicals such as benzoyl peroxide as in Kligman. In accordance with the present invention, it is used with two other agents that have healing or non-irritating properties. Alternatively, salicylic acid can be used in the present topical formulation at lower levels (i.e., less than 8% by weight) in combination with another keratolytic agent. For example, in one embodiment of this invention the keratolytic component of the acne formulation comprises about 2% by weight salicylic acid and from about 5 to about 25% benzoic acid.

The keratolytic agents are used in the present compositions at a level of about 8% to about 30% by weight, preferably about 9% to about 15% by weight, and most preferably about 10% by weight.

Astringent agents function in the present compositions to aid in the emptying of the engorged follicle, to reduce swelling in and around the affected follicle, and to contract other large skin pores so that acne bacteria present on the skin's surface are less likely to enter such pores. Representative astringents useful in this invention include zinc oxide, aluminum chloride, alum, aluminum acetate, calamine, zinc acetate, zinc sulfate, and zinc chloride. Zinc oxide is a preferred astringent because it is readily available and works well in the presence of the other active ingredients. Astringent materials are used in the present compositions in an amount ranging from about 2% to about 20% by weight, preferably about 5% to about 15% by weight.

The present composition also includes a mild anti-inflammatory agent. This is in contrast to current acne therapy which advises against treatment with strong anti-inflammatory agents. The anti-inflammatory agent used must have sufficient potency to prevent the host from reacting to the irritating contents of the follicle. At the same time, its potency should not be so strong that the area of the body with which the anti-inflammatory agent is in contact is left defenseless against other potentially more harmful reactants than those discharged from the plugged follicles. Preferred anti-inflammatory agents useful in this invention are non-steroidal compounds including anti-inflammatory amino acids, amino sugars, and non-steroidal prostaglandin synthesase inhibitors such as ibuprofen.

Amino acids known to have anti-inflammatory activity include cysteine, L-tryptophan, valine, alanine, glycine, glutamine, aspartic acid, S-methylcysteine, phenylalanine and leucine. Cysteamine is also recognized to exhibit anti-inflammatory activity. Exemplary of anti-inflammatory amino sugars are glucosamine, n-acetyl glucosamine, and mannosamine. Cysteine and glucosamine are preferred for use in the present compositions. If benzoyl peroxide or one of the other stronger comedolytics is selected for preparation of a composition in accordance with the present invention, a stronger anti-inflammatory agent such as 0.5% hydrocortisone or non-steroidal anti-inflammatory agents such as ibuprofen can be used advantageously.

Anti-inflammatory agents are typically present in this composition in an amount ranging from about 2% to about 30% by weight, preferably about 5% to about 15% by weight and most preferably about 10% by weight of the topical formulation inclusive of the carrier/vehicle base.

The active ingredients employed in this invention are preferably applied in combination formulated in a pharmaceutically acceptable vehicle for topical application. There are many commercially available gel, solution, lotion, emulsion, ointment or cream "bases" which can be used for the present topical formulations. Conventional liquid vehicles are water, alcohols such as ethanol, methanol, propanol and isopropanol; water alcohol solutions; or water-alcohol-polyalkylene glycol solutions, e.g., a vehicle containing 50% water, 30% ethanol and 20% propylene glycol. Commercially available cream or gel bases include DermabaseTM by Marcelle Division of Professional Cosmetics Corporation of Plattsburgh, New York and UnibaseTM by Parke-Davis Division of Warner-Lambert of Morris Plains, New Jersey. Other suitable vehicles for the present composition are described in U.S. Patents 4,355,028; 4,318,907; 4,126,681 and 4,244,928.

In a preferred embodiment of the present invention, the topical composition contains about 10% by weight cysteine or other anti-inflammatory amino acid or amino sugar, about 5% to about 10% by weight zinc oxide, about 10% by weight salicylic acid, and the remainder a suitable vehicle for topical application of said components. In another preferred embodiment the acne treatment formulation comprises about 2% to about 7% by weight salicylic acid; about 5 to about 30%, more preferably about 20%, by weight benzoic acid; about 5 to about 10%, more preferably about 5%, by weight zinc oxide; and about 10% by weight cysteine or other anti-inflammatory amino acid or amino sugar. Topical treatment is continued until the acne condition subsides.

The present invention is further illustrated by the following examples, none of which are to be construed as limiting the invention in any respect.

EXAMPLE 1

A preparation consisting of 10% salicylic acid, 10% zinc oxide, and 10% glucosamine in a commercial topical ointment base was used by two adult women (ages 34 and 38) having severe acne. The women had previously required constant physician care and the use of prescription drugs to keep their acne under control. After using a topical preparation of the above formulation for one week, marked improvement resulted for both women. After one month the women exhibited no active acne and did not require use of any other medication to keep their acne under control.

After two months, various modifications of the formulation were tried, including the use of zinc sulfate versus zinc oxide. Zinc sulfate was found to be a less effective astringent than zinc oxide in the present formulations.

Finally, the women were treated with a topical preparation consisting of 10% cysteine, 10% salicylic acid, and 5% zinc oxide in a cream base. This preparation resulted in the most beneficial effect. Using this latter preparation, the women were acne-free for the three month period of this study.

Substitution of a mixture of 2% salicylic acid and 20% benzoic acid for the "10% salicylic acid" component of the above topical preparations provides other effective acne treatment formulations in accordance with this invention.


EXAMPLE 2

Two adults (26 year old female and 32 year old male) having moderate acne used the formulation described in Example 1 and experienced significant improvement in their acne condition in one week. Their acne was maintained successfully under control with the second formulation of Example 1 (containing cysteine and 5% zinc oxide).


EXAMPLE 3

Two female college students, ages 21 and 22, having mild acne conditions tried various combinations of the compositions of Example 1. A preparation containing 10% glucosamine, 10% salicylic acid, and 5% zinc oxide was successful in maintaining their skin acne free.


EXAMPLE 4

Two teenagers (12 year-old girl with very mild acne and 15 year-old boy with moderate acne) were given a topical preparation of 10% salicylic acid, 10% glucosamine, and 10% zinc oxide blended in a topical ointment base. The subjects found the preparation to be the first effective treatment they had used. Previously, their use of preparations containing benzoyl peroxide had been found to be too irritating. Following partial remission of their acne condition, they switched to the second formulation consisting of 10% salicylic acid, 10% cysteine, and 5% zinc oxide in a vehicle for topical application and found it to be equally effective.

While the invention has been described with respect to specific materials, such materials are illustrative only. Numerous modifications and equivalents will be apparent to those of ordinary skill in this art without departing from the spirit of the invention.


**Claims**

1. A composition for treatment of acne comprising a keratolytic agent, an astringent, and an anti-inflammatory agent.

2. The composition of claim 1 wherein the keratolytic agent is selected from the group consisting of salicylic acid, retinoic acid, resorcinol, tretonin, sulfur, benzoic acid, urea and benzoyl peroxide.

3. The composition of claim 1 wherein the astringent is selected from the group consisting of pharmaceutically acceptable zinc oxide, zinc salts, and aluminum salts.

4. The composition of claim 1 wherein the anti-inflammatory agent is a non-steroidal anti-inflammatory agent.

5. The composition of claim 2 wherein the keratolytic agent is salicylic acid.

6. The composition of claim 3 wherein the astringent is selected from the group consisting of zinc oxide, aluminum chloride, alum, aluminum acetate, calamine, zinc acetate, zinc sulfate, and zinc chloride.

7. The composition of claim 6 wherein the astringent is zinc oxide.

8. The composition of claim 4 wherein the non-steriodal anti-inflammatory agent is selected from the group consisting of an anti-inflammatory amino acid or amino sugar, and prostaglandin synthesase inhibitors.

9. The composition of claim 8 wherein the amino acid is selected from the group consisting of cysteine and glucosamine.

10. The composition of claim 9 wherein the amino acid is cysteine.

11. The composition of claim 1 wherein the keratolytic agent is present in an amount ranging from about 8 to about 30 weight percent, the astringent is present in an amount ranging from about 2 to about 20 weight percent, and the anti-inflammatory agent is present in an amount ranging from about 2 to about 30 percent by weight.

12. The composition of claim 11 wherein the anti-inflammatory agent is cysteine or glucosamine, the astringent is zinc oxide, and the keratolytic agent is salicylic acid alone or in combination with a second keratolytic agent.

13. The composition of claim 12 wherein cysteine and salicylic acid are present at levels of about 10 percent by weight and the zinc oxide is present at a level of about 5 to about 10 percent by weight.

14. The composition of claim 12 wherein the keratolytic agent is a combination of salicylic acid and benzoic acid.

15. The composition of claim 14 containing about 2 to about 7% by weight salicylic acid, about 5 to about 30% benzoic acid, about 5 to about 10% by weight zinc oxide, about 10% by weight of cysteine or glucosamine and a pharmaceutically acceptable vehicle therefor.

16. The composition of claim 15 comprising about 2% by weight salicylic acid, about 20% by weight benzoic acid, about 5% by weight zinc oxide, about 10% by weight cysteine, and a pharmaceutically acceptable vehicle therefor.

17. A composition for treating acne consisting of about 8 to about 30 weight percent of a keratolytic agent, about 2 to about 20 weight percent of an astringent, about 2 to about 30 weight percent of an anti-inflammatory agent, and a pharmaceutically acceptable vehicle therefor.

18. A method for treating acne comprising applying to the affected area of skin an effective amount of

a keratolytic agent,

an astringent, and

an anti-inflammatory agent, in a pharmaceutically acceptable carrier therefor.

19. A method for treating acne comprising opening plugged skin follicles with a suitable keratolytic agent,

causing the follicle to contract with a suitable astringent, and

suppressing inflammatory response when the irritating contents of the follicle are discharged onto the skin with a mild anti-inflammatory agent.

20. The method of claim 18 wherein the keratolytic agent is salicylic acid, the astringent is zinc oxide, and the anit-inflammatory agent is cysteine or glucosamine.

Claims for the following Contracting States : ES and GR

1. A method of producing a composition for treatment of acne comprising mixing a keratolytic agent, an astrigent, and an anti-inflammatory agent.

2. The method of claim 1 wherein the keratolytic agent is selected from the group consisting of salicylic acid, retinoic acid, resorcinol, tretonin, sulfur, benzoic acid, urea and benzoyl peroxide.

3. The method of claim 1 wherein the astringent is selected from the group consisting of pharmaceutically acceptable zinc oxide, zinc salts, and aluminum salts.

4. The method of claim 1 wherein the anti-inflammatory agent is a non-steroidal anti-inflammatory agent.

5. The method of claim 2 wherein the keratolytic agent is salicylic acid.

6. The method of claim 3 wherein the astringent is selected from the group consisting of zinc oxide, aluminum chloride, alum, aluminum acetate, calamine, zinc acetate, zinc sulfate, and zinc chloride.

7. The method of claim 6 wherein the astringent is zinc oxide.

8. The method of claim 4 wherein the non-steroidal anti-inflammatory agent is selected from the group consisting of an anti-inflammatory amino acid or amino sugar, and prostaglandin synthesase inhibitors.

9. The method of claim 8 wherein the amino acid is selected from the group consisting of cysteine and glucosamine.

10. The method of claim 9 wherein the amino acid is cysteine.

11. The method of claim 1 wherein the keratolytic agent is present in an amount ranging from about 8 to about 30 weight percent, the astringent is present in an amount ranging from about 2 to about 20 weight percent, and the anti-inflammatory agent is present in an amount ranging from about 2 to about 30 percent by weight.

12. The method of claim 11 wherein the anti-inflammatory agent is cysteine or glucosamine, the astringent is zinc oxide, and the keratolytic agent is salicylic acid alone or in combination with a second keratolytic agent.

13. The method of claim 12 wherein cysteine and salicylic acid are present at levels of about 10 percent by weight and the zinc oxide is present at a level of about 5 to about 10 percent by weight.

14. The method of claim 12 wherein the keratolytic agent is a combination of salicylic acid and benzoic acid.

15. The method of claim 14 containing about 2 to about 7% by weight salicylic acid, about 5 to about 30% benzoic acid, about 5 to about 10% by weight zinc oxide, about 10% by weight of cysteine or glucosamine and a pharmaceutically acceptable vehicle therefor.

16. The method of claim 15 comprising about 2% by weight salicylic acid, about 20% by weight benzoic acid, about 5% by weight zinc oxide, about 10% by weight cysteine, and a pharmaceutically acceptable carrier therefor.

17. A method of producing a composition for treatment of acne consisting of mixing about 8 to about 30 weight percent of a keratolytic agent, about 2 to about 20 weight percent of an astringent, about 2 to about 30 weight percent of an anti-inflammatory agent, and a pharmaceutically acceptable vehicle therefor.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | WO-A-8 204 393 (RORER INTERNATIONAL) <br> * Amended claims 1,5 * <br> --- | 1,2,5 | A 61 K 33/30 <br> A 61 K 33/06 // <br> (A 61 K 33/30 |
| A | EP-A-0 004 686 (PROCTER & GAMBLE) <br> * Claims 1,2,4-8 * <br> --- | 1-3,5-8 | A 61 K 33:04 <br> A 61 K 31:60 |
| A | DRUG INFORMATION FULL TEXT 01998828 <br> AHFS, no. 84.28, American Hospital <br> Formulary Service, Monograph: Sulfur: <br> "Keratolytic Agents" <br> * Acné, additional text: uses * <br> ----- | 1,2,5 | A 61 K 31:19 <br> A 61 K 31:195) <br> (A 61 K 33/06 <br> A 61 K 33:04 <br> A 61 K 31:60 <br> A 61 K 31:19 <br> A 61 K 31:195) |

TECHNICAL FIELDS SEARCHED (Int. Cl.4)

A 61 K

The present search report has been drawn up for all claims

| Place of search | Date of completion of the search | Examiner |
|---|---|---|
| THE HAGUE | 14-06-1988 | PEETERS J.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................

& : member of the same patent family, corresponding document

EPO FORM 1503 03.82 (P0401)